# EUROPEAN PATENT SPECIFICATION

(11) **EP 2 556 826 B1**
(45) Date of publication and mention of the grant of the patent: **21.09.2016**
(21) Application number: 12179426.7
(22) Date of filing: 06.08.2012
(51) Int. Cl.: A61K 9/50, A61K 31/554

(54) **Modified-release multiparticulate formulation of diltiazem HCL**
Mehrteilchenformulierung von Diltiazem-HCL mit modifizierter Freisetzung
Formulation multiparticulaire à libération modifiée de diltiazem HCL

(30) Priority: 08.08.2011 IT FI20110172
(43) Date of publication of application: 13.02.2013
(73) Proprietor: Valpharma S.p.A., 47899 Serravalle (SM)
(72) Inventor: Valducci, Roberto, 47039 Savignano sul Rubicone (IT); Alighieri, Tiziano, 47900 Rimini (IT); Avanessian, Serozh, 47827 Villa Verucchio (IT)
(74) Representative: Valenza, Silvia

(56) References cited:
- EP-A1- 1 125 586
- US-A- 5 670 172

## Description

### Field of the invention

The present invention relates to pharmaceutical formulations, in particular to formulations of diltiazem HCl.

### Prior art

The concept of modifying the contraction of cardiac muscle and smooth muscle by blocking the transportation of calcium ions is the basis for creating a vast and heterogenous group of drugs named as a whole calcium antagonists.

These drugs, which are also called calcium entry blockers, disactivate the interrupter of contraction, both in the myocardium and in the smooth muscle, by inhibiting passage across the cell membrane.

The resulting effect is relaxation above all of the arterial bed, as well as inotropic and negative chronotropic effects on the heart.

Chemically, the molecules capable of activating calcium channel blockade belong to various classes of chemical compounds, some of which have been established reference drugs for some time:
- phenylacetonitrile derivatives, such as verapamil
- dihydropiridine derivatives, such as nifedipine
- benzothiazepine, such as diltiazem.

Verapamil and diltiazem exert a slowing of the sinoatrial node rate and depression of atrioventricular contraction, effects attributed to a slowing of the recovery capacity of the channels.

In practice, the time necessary for the channel to reacquire its activation capacity is increased.

Calcium antagonists are widely used in the treatment of arrhythmia, anginal states, and hypertension.

In particular diltiazem, which produces a 5-10 mmHg reduction in the arterial blood pressure following oral administration, is used for the treatment of coronary insufficiency, anginal states, for postinfarction therapy, and to treat hypertension. Following oral administration, absorption is rapid and total, with manifest effects within 30-60 minutes and considerable first-pass metabolism in the liver, which reduces the bioavailability to around 40%.

Interindividual variability is significant.

The average dosages vary from 180 to 360 mg/day, subdivided on average into 3-4 administrations, repeated over the course of the day.

Since most of these patients are usually on multifactorial therapies, much attention has been paid to limiting the inconvenience due to repeated and sustained administrations.

Various formulations of modified-release diltiazem HCl are known from the prior art. Many of these formulations are of the multiparticulate type, the micro-granules of which comprise inert nuclei to which the active principle has been applied and then appropriately coated with controlled-release membranes (US 5344657, WO2005/016317, WO 97/23219, US 2003/0003149, US 7067151, WO 00/04883, WO 96/29992, US 5622716, WO 93/00093).

US 5670172 describes pharmaceutical formulations containing controlled-released diltiazem, said formulations comprising a core of diltiazem and a spheronising agent, said core being coated with a layer which enables controlled release of diltiazem.

EP 1 125586 describes tablets containing controlled-release diltiazem wherein the outer coating is made of methylcellulose and stearic acid.

The technical problem not yet resolved in the prior art is that of having available a formulation of diltiazem HCl which, at an appropriate dose and minimum volume, may be given as a single daily administration while guaranteeing, by means of appropriate sustained release, therapeutic levels of the drug throughout the course of the day.

### Summary of the invention

The present invention resolves the above-mentioned products by means of granules comprising diltiazem HCl, said granules being characterised by:
a. an inner core of diltiazem HCl having an average diameter of 500-710 µm;
b. a layer of diltiazem HCl spherically surrounding the core, applied to the core by spraying a suspension containing powdered diltiazem HCl;
c. an external coating which is capable of providing a sustained release of diltiazem HCl;
said granules overall having an average diameter of 1000-1200 microns and a diltiazem HCl titer greater than or equal to 90% by weight.

The granules as described above are adapted to the preparation of sustained-release multiparticulate pharmaceutical formulations comprising diltiazem HCl.

A further object of the invention is therefore a pharmaceutical formulation comprising diltiazem HCl, said pharmaceutical formulation comprising the granules as described above.

The present invention is characterised by the following advantages:
- the multiparticulate formulation (i.e. comprising the granules according to the invention) is excellent for stabilising gastrointestinal transit;
- the particularly high diltiazem HCl concentration of the granules enables a correct dosage by administration of a minimum volume;
- the sustained release profile of the granules of the invention is suitable for maintaining optimal therapeutic levels with the administration of 1 dose/day.

A further object of the present invention is a process for preparing the granules of the invention, said process comprising the following steps:
i) preparing cores a) of diltiazem HCl using granulation, extrusion, drying and screening;
ii) enlarging/rounding the cores obtained from step i) by applying layer b) by spraying a suspension of powdered diltiazem HCl;
iii) external coating the enlarged granules obtained from step ii) with a coating adapted to provide a sustained release of diltiazem HCl.

### Detailed description of the invention

The core a) is obtained by granulation, extrusion, drying and screening.

The core a) essentially consists of at least 95% by weight of diltiazem HCl, up to 5% by weight of suitable binding agents and possibly negligible amounts of residues of processing solvents; wherein the given percentages are calculated on the total weight of the core.

The layer b) essentially consists of at least 95% of diltiazem HCl, up to 5% by weight of suitable binding agents and possibly negligible amounts of residues of processing solvents; wherein the given percentages are calculated on the total weight of the layer b).

The layer b) applied to the core a) enlarges and rounds the granules.

In the granules according to the invention, the whole of the core a) and layer b) present a practically spherical form of average diameters of 1000-1200 microns, a diltiazem HCl titer greater than or equal to 95% by weight, more preferably greater than or equal to 97%.

The nucleus a) has a higher density than the layer b). The different density of the two layers has been observed and evaluated by scanning a section of a granule with an electron microscope.

The core a) is preferably obtained by means of the following steps:
i-1) granulating powdered diltiazem HCl in the presence of suitable solvents;
i-2) extruding the slurry obtained from step i-1) through a 1300 micron screen;
i-3) drying the pellets obtained from step i-2);
i-4) by a vibrating screen, selecting the pellets obtained from step i-3 through a screen with holes of 500-710 microns in diameter.

Granulation i-1) preferably takes place by adding to the powdered diltiazem HCl, for example in a high-speed granulator, a solution of a binding agent in a suitable solvent, preferably a mixture of water and an alcoholic solvent (for example ethanol).

Extrusion i-2) preferably takes place at ambient temperature (20-25°C) by forcing through a screen with holes of suitable sizes (for example 1300 micron).

The layer b) surrounding the granule core is applied (see step ii of the process mentioned above) by spraying a suspension containing powdered diltiazem HCl, said powder preferably being suspended in an alcoholic solvent, and yet more preferably the alcoholic solvent used being ethanol. The suspension may furthermore comprise suitable binding agents.

The diltiazem HCl powder used for applying the layer b) may advantageously comprise, for obvious reasons of economy and optimisation of the amount of active principle, the granules rejected during screening i-4) and then reduced to powder by micronisation.

Binding agents for granulation and spraying are preferably povidone, hydroxypropyl methylcellulose (HMPC), polyethylene glycol (PEG), sucrose, lactose or other similar sugars.

The coating c) adapted to provide a sustained release of diltiazem HCl is a thin layer or film, and preferably comprises
- a polymer selected from ethyl cellulose, methyl cellulose, hydroxypropyl methyl cellulose, hydroxyethyl cellulose, hydroxypropyl cellulose, hydroxybutyl cellulose, sodium carboxymethyl cellulose, hydroxypropyl methyl cellulose phthalate, cellulose acetate phthalate, polyvinyl acetate, polyvinylpyrrolidone, polyvinylacetophtalate and polyethylene glycol;
   in combination with
- a lipophilic compound selected from paraffin and fatty acids with 12 to 20 carbon atoms.

Yet more preferably, the coating c) comprises methyl cellulose and stearic acid. The coating may further comprise other pharmaceutically acceptable excipients or diluents, for example talcum.

Application of the coating may take place by the usual methods with the use of suitable solvents, such as acetone.

The granules according to the invention exhibited a release which is sustained for 24 hours. Such a release is therefore particularly adapted to the administration of daily doses of the drug.

The preferred pharmaceutical formulations according to the invention may be tablets, capsules or oral suspensions comprising the granules according to the invention.

The present invention will be better understood in the light of the following practicable examples.

### EXPERIMENTAL PART

### Example 1

The diltiazem HCl powder is introduced into the high-speed granulator, and the solution containing povidone in ethanol/water is begun to be added in the following quantities:

| | | |
|---|---|---|
| diltiazem HCl | 4000 g. | |
| Povidone | 112 g. | 12.73% |
| BG ethanol | 448 g. | 50.90% |
| Purified water | 320 g. | 36.37% |

After adding to the diltiazem powder the whole of the solution previously described, the slurry obtained is forced through a 1300 micron screen. The granules thus obtained are dried in a drying cabinet.

At the end of drying, the granules thus obtained are graded by means of a vibrating screen provided with screens having holes of 500 and 710 micron diameters. Granules obtained which are outside these limits are micronised and applied to conforming granules, which constitute the nuclei.

### Example 2

A portion of the nuclei produced in Example 1 are introduced into a coating pan and enlarged/rounded by spraying an alcoholic suspension containing powdered diltiazem HCl, micronised granules (rejected as being over- or under-sized) obtained in Example 1 and povidone in these quantities:

| | |
|---|---|
| Cores of diltiazem HCl | 200 g. |
| Powdered diltiazem HCl | 500 g. |
| Powdered micronised granules | 800 g. |
| Povidone | 53 g. |
| BG ethanol | 5412 g. |

**1000 g of the pellets obtained are then coated in a coating pan with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcu | 18 g. | 2.57% |

**Operating conditions:**

| | |
|---|---|
| Inlet air temperature: | 45 °C |
| Outlet air temperature: | 25-30 °C |
| Temperature of nuclei: | 25-30 °C |

**Analysis of these pellets produced the following results: Yields of 0.1 M HCl**

| | |
|---|---|
| 1^{st} hour | 9.9% |
| 4^{th} hour | 25.0% |
| 8^{th} hour | 51.2% |
| 16^{th} hour | 88.1% |
| 24^{th} hour | 99.9% |

### Example 3

A portion of the nuclei produced in Example 1 is introduced into a coating pan and enlarged/rounded by applying powdered diltiazem HCl, micronised granules (rejected as being over- or under-sized) obtained in Example 1 by washing the above-mentioned granules with an alcoholic solution of povidone, in these quantities:

| | | |
|---|---|---|
| diltiazem HCl granules | 200 g. | |
| Powdered diltiazem HCl | 500 g. | |
| Powdered micronised granules | 800 g. | |
| Povidone | 53 g. | 17.9% |
| BG ethanol | 243 g. | 82.1% |

**1000 g of the pellets obtained are then coated in a coating pan with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcum | 18 g. | 2.57% |

**Operating conditions:**

| | |
|---|---|
| Inlet air temperature: | 45 °C |
| Outlet air temperature: | 25-30 °C |
| Temperature of nuclei: | 25-30 °C |

**Analysis of these pellets produced the following results: Yields of 0.1 M HCl**

| | |
|---|---|
| 1^{st} hour | 13.6% |
| 4^{th} hour | 28.9% |
| 8^{th} hour | 61.6% |
| 16^{th} hour | 81.3% |
| 24^{th} hour | 99.7% |

### Example 4

A portion of the nuclei produced in Example 1 is introduced into a fluidised bed provided with a rotor insert and enlarged/rounded by spraying an alcoholic suspension containing powdered diltiazem HCl, micronised granules (rejected as being over- or under-sized) obtained in Example 1 and povidone in these quantities:

| | |
|---|---|
| diltiazem HCl granules | 200 g. |
| Powdered diltiazem HCl | 500 g. |
| Powdered micronised granules | 800 g. |
| Povidone | 53 g. |
| BG ethanol | 5412 g. |

**1000 g of the pellets obtained are then coated in a fluidised bed provided with a wurster insert with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcum | 18 g. | 2.57% |

**Operating conditions:**

| | |
|---|---|
| Inlet air temperature: | 45 °C |
| Outlet air temperature: | 25-30 °C |
| Temperature of nuclei: | 25-30 °C |

**Analysis of these pellets produced the following results: Yields of 0.1 M HCl**

| | |
|---|---|
| 1^{st} hour | 12.0% |
| 4^{th} hour | 24.7% |
| 8^{th} hour | 52.1% |
| 16^{th} hour | 79.5% |
| 24^{th} hour | 92.3% |

### Example 5

A portion of the granules produced in Example 1 is introduced into a fluidised bed provided with a rotor insert and enlarged/rounded by washing the above-mentioned granules with an alcoholic solution of povidone, in these quantities:

| | | |
|---|---|---|
| diltiazem HCl granules | 200 g. | |
| Powdered diltiazem HCl | 500 g. | |
| Powdered micronised granules | 800 g. | |
| Povidone | 53 g. | 17.9% |
| BG ethanol | 243 g. | 82.1% |

**1000 g of the pellets obtained are then coated in a fluidised bed provided with a wurster insert with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcum | 18 g. | 2.57% |

**Operating conditions:**

| | |
|---|---|
| Inlet air temperature: | 40 °C |
| Outlet air temperature: | 25-30 °C |
| Nuclei temperature: | 25-30 °C |

**Analysis of these pellets produced the following results: Yields of 0.1 M HCl**

| | |
|---|---|
| 1^{st} hour | 12.0% |
| 4^{th} hour | 24.2% |
| 8^{th} hour | 54.1% |
| 16^{th} hour | 77.6% |
| 24^{th} hour | 96.2% |

### Example 6

The diltiazem HCl powder is introduced into a coating pan, and the solution of povidone in ethanol/water is begun to be sprayed in the following quantities:

| | | |
|---|---|---|
| diltiazem HCl | 4000 g. | |
| Povidone | 112 g. | 12.73% |
| BG ethanol | 448 g. | 50.90% |
| Acqua purificata | 320 g. | 36.37% |

After spraying the whole of the solution described previously onto the diltiazem powder, the slurry obtained is forced through a 1300 micron screen. The granules thus obtained are dried in a drying cabinet.

At the end of drying, the granules thus obtained are graded by means of a vibrating screen provided with screens having holes of 500 and 710 micron diameters. Granules obtained which are outside these limits are micronised and applied to conforming granules.

### Example 7

A portion of the granules produced in Example 6 are introduced into a coating pan and enlarged/rounded by spraying an alcoholic suspension containing powdered diltiazem HCl, micronised granules (rejected as being over- or under-sized) obtained in Example 1 and povidone in these quantities:

| | |
|---|---|
| Nuclei of diltiazem HCl | 200 g. |
| Powdered diltiazem HCl | 500 g. |
| Powdered micronised granules | 800 g. |
| Povidone | 53 g. |
| BG ethanol | 5412 g. |

**1000 g of the pellets obtained are then coated in a coating pan with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcum | 18 g. | 2.57% |

**Operating conditions:**

| | |
|---|---|
| Inlet air temperature: | 40 °C |
| Outlet air temperature: | 25-30 °C |
| Nuclei temperature: | 25-30 °C |

**Analysis of these pellets produced the following results: Yields of 0.1 M HCl**

| | |
|---|---|
| 1^{st} hour | 10.1% |
| 4^{th} hour | 28.7% |
| 8^{th} hour | 2860.1% |
| 16^{th} hour | 86.2% |
| 24^{th} hour | 98.7% |

### Example 8

A portion of the granules produced in Example 6 is introduced into a coating pan and enlarged/rounded by applying powdered diltiazem HCl, powdered micronised granules (rejected as being over- or under-sized) obtained in Example 6 by washing the above-mentioned granules with an alcoholic solution of povidone, in these quantities:

| | | |
|---|---|---|
| Granules of diltiazem HCl | 200 g. | |
| Powdered diltiazem HCl | 500 g. | |
| Powdered micronised granules | 800 g. | |
| Povidone | 53 g. | 17.9% |
| BG ethanol | 243 g. | 82.1% |

**1000 g of the pellets obtained are then coated in a coating pan with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcum | 18 g. | 2.57% |

**Operating conditions:**

| | |
|---|---|
| Inlet air temperature: | 40 °C |
| Outlet air temperature: | 25-30 °C |
| Nuclei temperature: | 25-30 °C |

**Analysis of these pellets produced the following results: Yields of 0.1 M HCl**

| | |
|---|---|
| 1^{st} hour | 11.2% |
| 4^{th} hour | 27.7% |
| 8^{th} hour | 67.6% |
| 16^{th} hour | 89.7% |
| 24^{th} hour | 101.3% |

### Example 9

A portion of the nuclei produced in Example 6 is introduced into a fluidised bed provided with a rotor insert and enlarged/rounded by spraying an alcoholic suspension containing powdered diltiazem HCl, micronised granules (rejected as being over- or under-sized) obtained in Example 6 and povidone in these quantities

| | |
|---|---|
| diltiazem HCl granules | 200 g. |
| Powdered diltiazem HCl | 500 g. |
| Powdered micronised granules | 800 g. |
| Povidone | 53 g. |
| BG ethanol | 5412 g |

**1000 g of the pellets obtained are then coated in a fluidised bed provided with a wurster insert with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcum | 18 g. | 2.57% |

**Operating conditions:**

| | |
|---|---|
| Inlet air temperature: | 40 °C |
| Outlet air temperature: | 25-30 °C |
| Nuclei temperature: | 25-30 °C |

**Analysis of these pellets produced the following results: Yields of 0.1 M HCl**

| | |
|---|---|
| 1^{st} hour | 9.9% |
| 4^{th} hour | 23.8% |
| 8^{th} hour | 55.1% |
| 16^{th} hour | 78.7% |
| 24^{th} hour | 98.4% |

### Example 10

A portion of the granules produced in Example 6 is introduced into a fluidised bed provided with a rotor insert and enlarged/rounded by applying powdered diltiazem HCl, micronised granules (rejected as being over- or under-sized) obtained in Example 6, by washing the above-mentioned granules with an alcoholic solution of povidone, in these quantities:

| | | |
|---|---|---|
| diltiazem HCl granules | 200 g. | |
| Powdered diltiazem HCl | 500 g. | |
| Powdered micronised granules | 800 g. | |
| Povidone | 53 g. | 17.9% |
| BG ethanol | 243 g. | 82.1% |

**1000 g of the pellets obtained are then coated in a fluidised bed provided with a wurster insert with a solution of ethyl cellulose/stearic acid of the following composition:**

| | | |
|---|---|---|
| Ethyl cellulose | 21 g. | 3% |
| Stearic acid | 2.1 g. | 0.3% |
| Acetone | 658.9 g. | 94.13% |
| Talcum | 18 g. | 2.57% |

**Operating conditions:**

| | |
|---|---|
| Inlet air temperature: | 40 °C |
| Outlet air temperature: | 25-30 °C |
| Nuclei temperature: | 25-30 °C |

**Analysis of these pellets produced the following results: Yields of 0.1 M HCl**

| | |
|---|---|
| 1^{st} hour | 10.0% |
| 4^{th} hour | 25.9% |
| 8^{th} hour | 57.7% |
| 16^{th} hour | 83.6% |
| 24^{th} hour | 102.3% |

### Example 11

The powdered diltiazem HCl is introduced into a coating pan, and the solution of povidone in ethanol/water is begun to be sprayed in the following quantities:

| | | |
|---|---|---|
| diltiazem HCl | 10000 g. | |
| Povidone | 280 g. | 12.73% |
| BG ethanol | 1120 g. | 50.90% |
| Purified water | 800 g. | 36.37% |

After spraying the whole of the solution previously described on to the powdered diltiazem, the slurry obtained is forced through a 1300-micron screen. The granules thus obtained are dried in a drying cabinet.

At the end of drying, the granules thus obtained are graded by means of a vibrating screen provided with screens having holes of 500 and 710 micron diameters. Granules obtained which are outside these limits are micronised and applied in powder form to conforming granules (together with powdered diltiazem as such).

All the granules obtained which conform to the screened size are introduced into the coating pan, and are enlarged/rounded by applying powdered diltiazemHCl, powdered micronised granules (outside the screening limits) by washing the granules in an alcoholic solution of povidone, in these quantities:

| | | |
|---|---|---|
| diltiazem HCl granules | 2200 g. | |
| Powdered diltiazem HCl | 5000 g. | |
| Powdered micronised granules | 8000 g. | |
| Povidone | 528 g. | 17.9% |
| BG ethanol | 2422 g. | 82.1% |

**In a fluidised bed equipped with a wurster insert, the pellets obtained (15.0 kg) are then coated dissolution of ethyl cellulose/stearic acid of the following composition:**

| | | |
|---|---|---|
| Ethyl cellulose | 312 g. | 3 % |
| Stearic acid | 31.2 g. | 0.3 % |
| Acetone | 9791.8 g. | 94.15 % |
| Talcum | 265 g. | 2.55 % |

**Operating conditions:**

| | |
|---|---|
| Inlet air temperature: | 45 °C |
| Outlet air temperature: | 25-30 °C |
| Nuclei temperature: | 25-30 °C |

**Analysis of these pellets produced the following results: Yields of 0.1 M HCl**

| | |
|---|---|
| 1^{st} hour | 12.9% |
| 4^{th} hour | 26.2% |
| 8^{th} hour | 57.2% |
| 16^{th} hour | 84.1% |
| 24^{th} hour | 95.3% |

## Claims

1. Granules comprising diltiazem HCl, said granules being **characterised by**:
a) an inner core of diltiazem HCl having an average diameter of 500-710 microns;
b) a layer of diltiazem HCl spherically surrounding the core, applied to the core by spraying a suspension containing powdered diltiazem HCl;
c) an external coating which is capable of providing a sustained release of diltiazem HCl;
said granules generally having an average diameter of 1000-1200 microns and a diltiazem HCl titer greater than or equal to 90% by weight.

2. Granules according to claim 1 wherein the core a) is obtained by granulation, extrusion, drying and screening.

3. Granules according to any one of claims 1-2, wherein the core a) consists of at least 95% by weight of diltiazem HCl, up to 5% by weight of suitable binding agents and possibly negligible amounts of residuals of processing solvents; wherein the given percentages is calculated on the total weight of the core.

4. Granules according to any one of claims 1-3, wherein the layer b) consists for at least 95% of diltiazem HCl, up to 5% by weight of suitable binding agents and possibly negligible amounts of residuals of processing solvents; wherein the given percentages is calculated on the total weight of the layer b).

5. Granules according to any one of claims 3-4, wherein said binding agents are selected from povidone, hydroxypropyl methylcellulose (HPMC), polyethylene glycol (PEG), sucrose, lactose or other similar sugars.

6. Granules according to any one of claims 1-5, wherein the coating c) adapted to confer a sustained release of diltiazem HCl is a thin layer or film, and comprises
- a polymer selected from ethyl cellulose, methyl cellulose, hydroxypropyl methyl cellulose, hydroxyethyl cellulose, hydroxypropyl cellulose, hydroxybutyl cellulose, sodium carboxymethyl cellulose, hydroxypropyl methyl cellulose phthalate, cellulose acetate phthalate, polyvinyl acetate, polyvinylpyrrolidone, polyvinylacetophtalate and polyethylene glycol;
in combination with
- a lipophilic compound selected from paraffin and fatty acids with 12 to 20 carbon atoms.

7. A process for preparing granules according to any one of claims 1-6, said process comprising the following steps:
i) preparing cores a) of diltiazem HCl using granulation, extrusion, drying and screening;
ii) enlarging/rounding the cores obtained from step i) by applying layer b) by spraying a suspension of powdered diltiazem HCl;
iii) outer covering the enlarged granules obtained from step ii) with a coating adapted to provide a sustained release of diltiazem HCl.

8. A process according to claim 7, wherein the step i) occurs through the following steps of:
i-1) granulating powdered diltiazem HCl in the presence of suitable solvents;
i-2) extruding the slurry obtained from step i-1) by means of a 1300 micron screen;
i-3) drying the pellets obtained from step i-2);
i-4) by a vibrating screen, selecting the pellets obtained from step i-3 by means of a screen with holes of 500-710 microns in diameter.

9. A pharmaceutical formulation comprising the granules according to claims 1-6.

## Patentansprüche

1. Diltiazem HCl enthaltendes Granulat, **gekennzeichnet durch**
a) einen inneren Kern aus Diltiazem HCl mit einem mittleren Durchmesser von 500-710 µm;
b) eine Diltiazem HCl Schicht, die den Kern sphärisch umgibt und auf den Kern mit einer pulverisiertes Diltiazem HCl enthaltenden Suspension aufgesprüht wird;
c) eine äußere Beschichtung, die eine kontinuierliche Diltiazem HCl Abgabe ermöglicht; wobei die Körnchen im Allgemeinen einen mittleren Durchmesser von 1000-1200 µm und einen Diltiazem HCl-Titer größer oder gleich 90 Gewichts-% aufweisen.

2. Granulat nach Anspruch 1, wobei der Kern a) durch Granulation, Extrusion, Trocknen und Sieben erhalten wird.

3. Granulat nach einem der Ansprüche 1-2, wobei der ein Kern a) aus mindestens 95 Gewichts-% Diltiazem HCl, aus bis zu 5 Gewichts-% geeigneten Bindemitteln und gegebenenfalls vernachlässigbaren Mengen an Verarbeitungslösungsmittelrückständen besteht; wobei die angegebenen Prozentsätze durch das Gesamtgewicht des Kerns berechnet werden.

4. Granulat nach einem der Ansprüche 1-3, wobei die Schicht b) aus mindestens 95 Gewichts-% Diltiazem HCl, bis zu 5 Gewichts-% geeigneten Bindemitteln und gegebenenfalls vernachlässigbaren Mengen an Verarbeitungslösungsmittelrückständen besteht; wobei die angegebenen Prozentsätze durch das Gesamtgewicht der Schicht b) berechnet werden.

5. Granulat nach einem der Ansprüche 3-4, wobei die Bindemittel Povidon, Hydroxypropylmethylcellulose (HPMC), Polyethylenglykol (PEG), Saccharose, Lactose oder andere ähnliche Zucker sein können.

6. Granulat nach einem der Ansprüche 1-5, wobei die eine anhaltende Freisetzung von Diltiazem HCl ermöglichende Beschichtung c) eine dünne Schicht oder Folie darstellt, umfassend
- ein Polymer ausgewählt aus einer Gruppe umfassend Ethylcellulose, Methylcellulose, Hydroxypropylmethylcellulose, Hydroxyethylcellulose, Hydroxypropylcellulose, hydroxybutyl Cellulose, Natriumcarboxymethylcellulose, Hydroxypropylmethylcellulosephthalat, Celluloseacetatphthalat, Polyvinylacetat, Polyvinylpyrrolidon, polyvinylacetophtalate und Polyethylenglykol;
in Kombination mit
- einer lipophilen Verbindung ausgewählt aus Paraffin und Fettsäuren mit 12 bis 20 Kohlenstoffatomen.

7. Verfahren zur Herstellung von Granulat nach einem der Ansprüche 1-6, wobei das Verfahren folgende Schritte umfasst:
i) Herstellen von Kernen a) aus Diltiazem HCl unter Verwendung von Granulierung, Extrusion, Trocknen und Sieben;
ii) Vergrößern/Abrunden der Kerne aus Schritt i) durch Auftragen der Schicht b) durch Aufsprühen einer Suspension von pulverisiertem Diltiazem HCl;
iii) Überziehen des vergrößerten Granulats aus Schritt ii) mit einer Beschichtung, die eine kontinuierliche Abgabe von Diltiazem HCl ermöglicht.

8. Verfahren nach Anspruch 7, wobei der Schritt i) durch folgende Schritte gekennzeichnet ist:
i-1) Granulieren von pulverisiertem Diltiazem HCl in Gegenwart von geeigneten Lösungsmitteln;
i-2) Extrudieren der Suspension aus Schritt i-1) mittels eines 1300 µm Siebs;
i-3) Trocknen der Pellets aus Schritt i-2);
i-4) Auswählen der Pellets aus Schritt i-3 durch ein Rüttelsieb mit Öffnungen von 500 bis 710 µm Durchmesser.

9. Pharmazeutische Formulierung umfassend das Granulat nach den Ansprüchen 1-6.

## Revendications

1. Granules comprenant du diltiazem HCl, lesdites granules étant **caractérisées par** :
a) un noyau interne de diltiazem HCl ayant un diamètre moyen de 500 à 710 microns ;
b) une couche de diltiazem HCl entourant le noyau de manière sphérique, appliquée sur le noyau par pulvérisation d'une suspension contenant du diltiazem HCl en poudre ;
c) un revêtement externe qui est capable de fournir une libération prolongée de diltiazem HCl ;
lesdites granules ayant en général un diamètre moyen de 1 000 à 1 200 microns et un titre de diltiazem HCl supérieur ou égal à 90 % en poids.

2. Granules selon la revendication 1, dans lesquelles le noyau a) est obtenu par granulation, extrusion, séchage et tamisage.

3. Granules selon l'une quelconque des revendications 1-2, dans lesquelles le noyau a) est constitué d'au moins 95 % en poids de diltiazem HCl, jusqu'à 5 % en poids d'agents de liaison appropriés, et éventuellement de quantités négligeables de résidus de solvants de traitement ; dans lesquelles les pourcentages indiqués sont calculés par rapport au poids total du noyau.

4. Granules selon l'une quelconque des revendications 1 à 3, dans lesquelles la couche b) est constituée pour au moins 95 % du diltiazem HCl, jusqu'à 5 % en poids d'agents de liaison appropriés, et éventuellement de quantités négligeables de résidus de solvants de traitement ; dans lesquelles les pourcentages sont calculés par rapport au poids total de la couche b).

5. Granules selon l'une quelconque des revendications 3-4, dans lesquelles lesdits agents de liaison sont choisis parmi la povidone, l'hydroxypropylméthylcellulose (HPMC), du polyéthylène glycol (PEG), le saccharose, le lactose ou d'autres sucres similaires.

6. Granules selon l'une quelconque des revendications 1 à 5, dans lequel le revêtement c) adapté pour conférer une libération prolongée de diltiazem HCI est une couche ou un film mince, et comprend
- un polymère choisi parmi l'éthylcellulose, la méthylcellulose,l'hydroxypropylméthylcellulose, l'hydroxyéthylcellulose, l'hydroxypropylcellulose, l'hydroxybutylcellulose, la carboxyméthylcellulose sodique, le phtalate d'hydroxypropylméthylcellulose, le phtalate d'acétate de cellulose, l'acétate de polyvinyle, la polyvinylpyrrolidone, le polyvinylacétophtalate et le polyéthylène glycol ;
en combinaison avec
un composé lipophile choisi parmi de la paraffine et des acides gras avec 12 à 20 atomes de carbone.

7. Procédé de préparation de granules selon l'une quelconque des revendications 1 à 6, ledit procédé comprenant les étapes suivantes consistant à :
i) préparer des noyaux a) de diltiazem HCl en utilisant une granulation, une extrusion, un séchage et un tamisage ;
ii) agrandir/arrondir les noyaux obtenus à partir de l'étape i) par application de la couche b) par pulvérisation d'une suspension de diltiazem HCl en poudre ;
iii) recouvrir sur l'extérieur les granules agrandies obtenues à l'étape ii) avec un revêtement adapté pour fournir une libération prolongée de diltiazem HCl.

8. Procédé selon la revendication 7, dans lequel l'étape i) a lieu à travers les étapes suivantes consistant à :
i-1) granuler le diltiazem HCl en poudre en présence de solvants appropriés ;
i-2) extruder la pâte obtenue à l'étape i-1) au moyen d'un tamis de 1 300 microns ;
i-3) sécher les granules obtenues à l'étape i-2) ;
i-4) par l'intermédiaire d'un tamis vibrant, sélectionner les pastilles obtenues à l'étape i-3 au moyen d'un tamis avec des trous de 500 à 710 microns de diamètre.

9. Formulation pharmaceutique comprenant des granules selon les revendications 1 à 6.
